# FASCICULE DE BREVET EUROPEEN

(11) **EP 0 375 579 B1**
(45) Date de publication et mention de la délivrance du brevet: **14.07.1993**
(21) Numéro de dépôt: 89440134.8
(22) Date de dépôt: 13.12.1989
(51) Int. Cl.: A61M 35/00

(54) **Ustensile de badigeonnage à usage thérapeutique ou médical**
Gerät zum Auftragen in der Therapie oder Medizin
Pointing utensil for therapeutic or medical use

(30) Priorité: 20.12.1988 FR 8817051
(43) Date de publication de la demande: 27.06.1990
(73) Titulaire: Testelin, Gérard Marie, F-76016 Paris (FR)
(72) Inventeur: Testelin, Gérard Marie, F-76016 Paris (FR)
(74) Mandataire: Bossard, Jacques-René

(56) Documents cités:
- WO-A-87/06112
- DE-A- 3 416 003
- FR-A- 2 232 331
- US-A- 4 659 243
- US-A- 4 732 503

## Description

La présente invention a trait à un ustensile de badigeonnage de conception entièrement nouvelle destiné à la désinfection, décontamination, aseptisation de la peau avant toute intervention chirurgicale ou tout traitement nécessitant cette étape, laquelle est par exemple indispensable pour toute thérapie ou thérapeutique nécessitant une intervention cutanée et/ou transcutanée.

La pratique actuelle utilise des moyens et procédés inchangés depuis fort longtemps et qui se résument en un badigeonnage de la zone concernée de la peau par une compresse tenue par une pince. Cette opération d'apparence très simple se subdivise en réalité en plusieurs gestes plus ou moins complexes traduisant la nécessité impérative de travailler en milieu aseptique afin d'assurer la sécurité des patients et personnels soignants :
- la pince doit être prise dans une boîte stérile fermée,
- la compresse à monter sur la pince se trouve dans un tambour stérile qu'il faut ouvrir pour s'en servir,
- une cupule stérile sert à contenir ce liquide désinfectant, qui doit être versé avec les précautions d'usage.

Ces phases multiples sont au mieux synonymes de perte de temps, quand elle ne sont pas génératrices d'insécurité. Au surplus, un tel mode opératoire présente un certain nombre d'autres inconvénients : en premier lieu, les quantités de produits utilisées par ce système sont sans rapport avec le volume réel nécessaire, du fait d'un gaspillage intense naissant de cette manière de procéder. A titre d'exemple, une opération de prothèse de hanche nécessite actuellement à peu près quatre litre d'alcool, alors que la quantité réelle qu'une utilisation rigoureuse entraînerait est de l'ordre du quart de litre.

En second lieu, les compresses présentent souvent des surfaces très petites, et le badigeonnage peut par conséquent demander du temps si la partie à enduire est grande. Cette perte de temps peut s'accompagner d'une demande accrue en personnel lorsque l'opération est effectuée par plusieurs personnes afin d'aller plus vite.

En dernier lieu, il faut signaler qu'il n'est pas rare d'égarer la ou les compresses destinées au badigeonnage, ou encore qu'il arrive de les confondre avec les compresses dites opératoires. Cela a pour effet d'introduire un doute dans l'esprit du chirurgien, qui peut en effet légitimement se poser des questions quant à la nature des compresses qui sont restées dans la partie opérée du patient, sachant qu'une inversion aurait des conséquences dramatiques.

On a déjà proposé des ustensiles destinés à compenser au moins partiellement ces inconvénients, voir par exemple les documents US-A-4 659 243 et WO-A-87/06112. On peut résumer ces diverses caractéristiques de tels ustensiles de la manière suivante :

Ce sont des ustensiles de badigeonnage, destinés à l'application d'un produit pour la désinfection, la décontamination, l'aseptisation de la peau, ou tout traitement nécessitant une étape comparable, indispensable à une thérapie ou une thérapeutique nécessitant une intervention cutanée et/ou transcutanée, et se composant d'un rouleau d'application dont le moyeu creux, comportant des perforations permettant l'arrivée dudit produit à la surface dudit rouleau, et monté rotatif sur un arbre creux fixe et relié à un réservoir dudit produit, et comportant également des perforations.

Bien que ces ustensiles représentent effectivement un progrès par rapport aux techniques ne faisant intervenir que des compresses nouvelles évoquées plus haut, ils présentent encore des inconvénients liés à leur complexité et à leur prix.

En réponse à ces derniers inconvénients, le système de l'invention propose une conception originale, pratique d'utilisation, économique aussi bien dans sa fabrication que dans son mode opératoire usuel et procurant enfin une sécurité d'emploi sans commune mesure avec ce qui existant jusqu'alors.

Dans ce but, l'invention vise selon la revendication 1 un ustensile du type défini plus haut, mais dans lequel ledit rouleau est en forme de diabolo et les perforations respectives dudit moyeu creux et dudit arbre creux sont décalées les unes par rapport aux autres.

Au surplus, selon une autre caractéristique de l'invention, le système d'alimentation du rouleau est bifide, c'est à dire que la tubulure raccordant le réservoir au rouleau se divise en deux branches de manière que ladite alimentation se fasse par les deux extrémités dudit moyeu. Dans ce cas, ladite tubulure est raccordée auxdites branches par une plaque commune assurant la rigidité de l'ensemble du système.

Selon une autre caractéristique encore de l'invention, l'axe du rouleau est situé dans un plan différent de celui défini par ladite tubulure et le réservoir, de manière que la main de l'opérateur manoeuvrant l'appareil en la tenant par le réservoir demeure éloignée de la peau du patient.

Selon une possibilité, le liquide de désinfection sera contenu dans des cartouches de rechange qui sont constituées par le manche amovible lui-même qui s'adapte au tuyau. L'extrémité du tuyau coudé qui se fixe au manche creux, comporte alors au surplus un moyen de perforer la capsule de sécurité de la recharge de produit désinfectant, agissant au moment de l'opération de fixation.

Selon une autre possibilité, les cartouches de recharge sont constituées par des recharges indépendantes qui se logent dans le manche creux configuré pour elles et qui comportent aussi une capsule de sécurité placée dans la partie destinée à se fixer à l'extrémité du tuyau coudé. La fixation complète de ce dernier aura alors pour effet de supprimer la capsule, ou tout au moins son effet, et de permettre le passage du liquide dans le tuyau.

Les avantages de ce nouvel ustensile sont nombreux, allant d'un point de vue strictement technique aux facilités offertes par le mode opératoire, en passant par les arguments économiques et financiers.

En premier lieu, on peut noter que le fait de frotter la peau en badigeonnage pré-opératoire comporte un risque, celui de la germination potentielle cutanée, transportée d'un endroit à un autre par déplacement de la compresse utilisée. Il y a en effet une forte probabilité de sepsis potentielle due au frottement, qui doit être évitée dans la mesure du possible afin de protéger le patient contre d'éventuelles suppurations postérieures à l'opération. Car l'emploi de l'invention est double : dans un premier temps, la peau du patient est préparée la veille de l'intervention et dans un second temps un second temps, un badigeonnage prend place avant l'opération proprement dite.

En second lieu, l'ustensile de l'invention présente une grande facilité d'emploi, particulièrement grâce à l'utilisation de cartouches facilement interchangeables. Cette remarque vaut aussi pour les rouleaux, qu'on peut choisir de différentes tailles sans aucun problème d'adaptation puisque l'empreinte de fixation reste la même pour tous. En outre, il n'est plus possible de le confondre, du fait de son apparence, avec une compresse ou un élément de substitution utilisé au cours d'une intervention chirurgicale : les fonctions respectives de ces éléments sont différenciées d'un simple coup d'oeil.

En troisième lieu, l'emploi d'un tel dispositif conduit à une économie de temps et d'argent, ainsi qu'il ressort des avantages exposés ci-dessus. Il est à noter que cela permet d'éviter les salissures dues aux excès de produit désinfectant, et par conséquent aussi les nettoyages longs et coûteux du linge, de la table d'opération, de la salle d'opération, des vêtements chirurgicaux et de meubles ou matériels proches du site de cette phase essentielle pré-opératoire.

Enfin, l'appareil en tant que tel peut être complété par un manche réservoir supplémentaire destiné à augmenter la capacité du premier réservoir.

Parmi les particularités avantageuses de ce dispositif, on peut citer au surplus le fait que la simplicité de sa construction et son bon marché lui permet d'être aussi facilement stérilisable dans le cas d'une ré-utilisation, qu' à usage unique, jetable après utilisation.

On notera en passant que par rapport aux dispositifs à piston de manoeuvre proposés antérieurement, ce système présente outre sa simplicité de construction, une simplicité de manoeuvre, car il est évidemment difficile de pouvoir badigeonner la peau d'un patient tout en poussant en même temps un piston pour faire parvenir la substance active au rouleau.

Dans la partie suivante, l'invention va être décrite plus en détail, suivant un mode de réalisation préférentiel, en référence aux figures présentées en annexe, pour lesquelles :
- la figure 1 représente une vue d'ensemble d'une première réalisation du dispositif selon l'invention,
- la figure 2 montre une vue éclatée du manche de la figure 1,
- la figure 3 est une vue semblable à la figure 1 d'un second mode de réalisation de l'invention,
- la figure 4 est une vue par l'avant du dispositif de la figure 1.

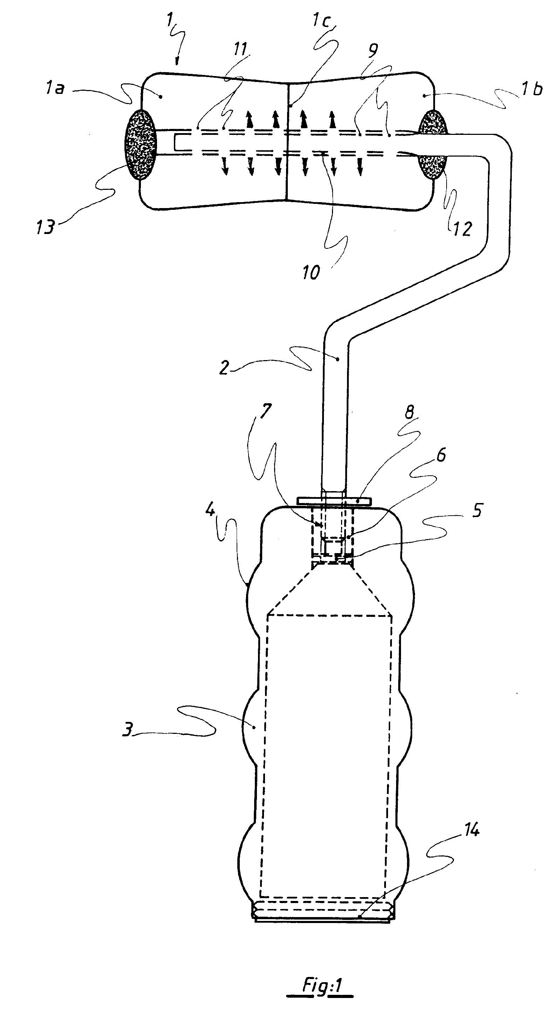

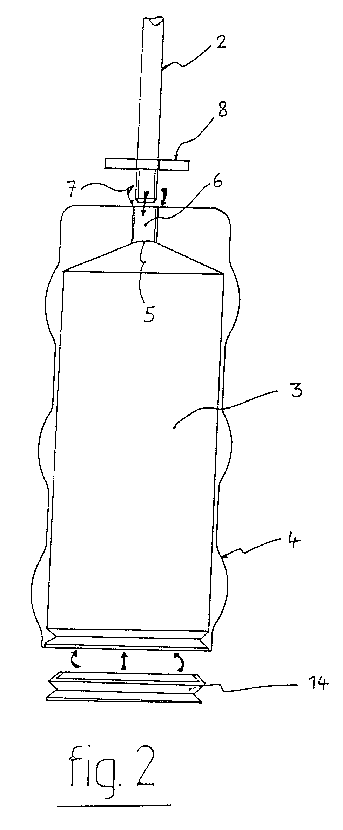

Selon la configuration représentée sur les figures 1 et 2, l'ustensile de l'invention se compose de trois parties essentielles, le rouleau (1) dans lequel s'emboîte le tuyau coudé (2) relié solidairement au manche (3).

Selon une particularité importante de l'invention, le rouleau 1 présente le forme d'un diabolo, c'est à dire de deux troncs de cônes 1a, 1b accolés par leurs petites bases suivant un petit cercle 1c. Une telle configuration qui n'a encore jamais été proposée, a pour avantage que ce rouleau permet un meilleur contact avec le corps humain, qui n'est jamais plan, notamment les membres et leurs interstices.

Le manche (3) est creux et sert de réservoir. Il est muni d'empreintes de préhension digitales (4) permettant une bonne stabilité au cours de l'emploi. Le fond du réservoir, au niveau de la liaison avec le tuyau coudé (2), comporte une capsule d'étanchéité (5) réalisée au cours du moulage. Elle assure l'étanchéité totale de l'ensemble, tant par son mode de fabrication que par la manière dont elle permet le passage du liquide dans le tuyau coudé (2). A l'autre extrémité du manche, l'obturation est réalisée à l'aide d'un capuchon clipsé (14) ou vissé qui permet le remplissage et assure une bonne étanchéité du réservoir lors d'un usage courant.

Le tuyau coudé est solidarisé au manche grâce à un filetage réalisé dans un conduit (6) placé immédiatement au-dessus de ladite capsule d'étanchéité. Le filetage (7) correspondant du tuyau permet la fixation dudit tuyau sur le manche. Le perçage s'effectue par rotation du tuyau coudé (2) dans le filetage du manche (3) : il suffit d'opérer un tour supplémentaire après que le bout du tuyau soit entré en contact avec la surface externe de la capsule (5) pour que l'ouverture pratiquée dans le réservoir permette le passage du liquide.

Une lèvre de sécurité (8) évite par ailleurs l'ouverture accidentelle du réservoir, avant utilisation pour badigeonnage, et permet la livraison de l'ensemble monté. Pour l'utilisation, il est nécessaire de compléter le vissage du manche, afin de casser la bague (8) circulaire ou semi-circulaire et assurer l'écoulement par perforation de la capsule d'étanchéité.

A l'autre extrémité du tuyau coudé creux, la partie introduite dans le rouleau (1) comporte des orifices de diffusion (9), par exemple de forme oblongue. Ceux-ci doivent être positionnés de façon à correspondre aux orifices de l'arbre creux du rouleau, dans lequel le tuyau coudé est fixé à cette extrémité. A cet effet, la forme des arbres peut être choisie légèrement tronconique de telle sorte que la fixation soit efficace et le déboîtement facile. Afin de respecter l'encombrement des rouleaux, qui peuvent avoir des diamètres variables, le tuyau prend une forme coudée laissant un espace suffisant pour l'insertion des diverses tailles de rouleaux.

Le rouleau mousse (1) est thermoformé, non altérable et inséré sur un moyeu (10), doté d'orifices oblongs (11) correspondant à ceux du tuyau coudé, mais décalés par rapport à eux. Ses caractéristiques dimensionnelles sont telles qu'il y a correspondance avec l'extrémité diffusante dudit tuyau, de sorte que la solidarisation de ces deux éléments est possible, sans que cela présente un caractère définitif. L'emboîtement est au contraire facilement réversible, par exemple pour un changement de rouleau. On retrouve ainsi la pente légère donnant la forme tronconique dans la partie femelle.

Deux bagues de maintien amovibles (12) et (13) servent de butées et évitent le déchaussement intempestif du rouleau, en même temps qu'elles servent d'auxiliaire à la fonction d'étanchéité.

Le réservoir (3) et le tuyau d'écoulement (2) peuvent par exemple être réalisés dans un matériau du type Lexan, ou tout produit similaire dont les caractéristiques sont compatibles avec les exigences médicales. Comme on l'a vu, différents modèles sont prévus, en étroite corrélation avec le type d'application envisagé. Ainsi, les rouleaux pourront être réalisés avec des longueurs de 3, 5, et 8 cm avec des diamètres respectifs de 15, 30 et 50 mm.

De même, les capacités usuelles prévues pour les manches réservoirs peuvent varier de 30 cm³ à 250 cm³, selon l'usage. Toutes les combinaisons sont possibles, par exemple l'adaptation d'un grand réservoir à un très petit rouleau, ou l'inverse.

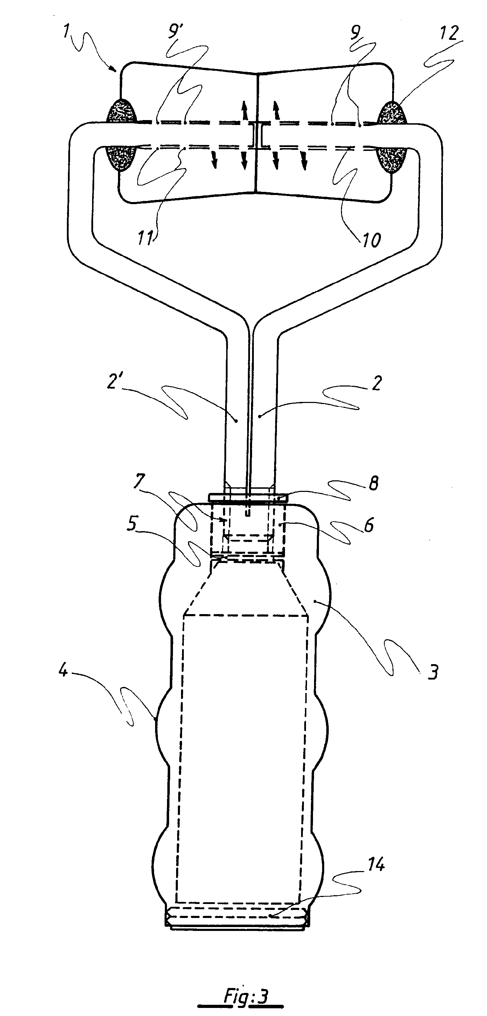

Dans la variante illustrée dans la figure 3, au lieu d'un tubulure 2 unique se terminant par un tuyau 9 enfoncé dans toute la longueur du moyeu 10, le rouleau 1 est alimenté sur ses deux côtés par deux tubulures symétriques 2 et 2' se terminant par deux tuyaux 9 et 9' se rejoignant vers le centre du moyeu 10. De cette manière, l'alimentation du rouleau est plus régulière, ce qui a son importance dans le cas de rouleaux de grande longueur.

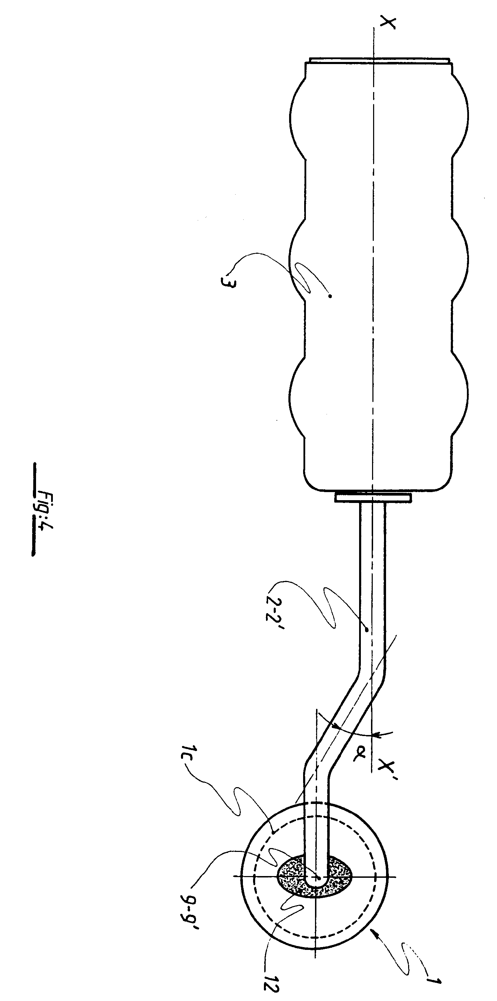

Enfin, la figure 4 fait ressortir le fait que, de préférence, l'axe XX' commun au moyeu 10 et au tuyau 9 est incliné par rapport au plan dans lequel est situé la tubulure 2.

Cette inclinaison d'un angle α de l'ordre de 20-30°, a pour avantage de faciliter la manoeuvre de l'appareil, tout en maintenant le réservoir 3 éloigné du corps du patient.

Il est à noter que l'ustensile de l'invention offre une très grande souplesse d'emploi, car il combine différents modes d'alimentation en liquide thérapeutique avec différents modes d'application dudit liquide sur la peau. Ainsi, on peut soit changer de réservoir si les conditions d'hygiène l'exigent, soit procéder au remplissage du même réservoir. Dans les deux cas, on peut choisir toutes formes de rouleaux en fonction du liquide et de sa fonction thérapeutique.

Comme on l'a vu, cet ustensile présente essentiellement un avantage médical déterminant dans la mesure où il permet d'éviter tout frottement sur la peau et d'empêcher par conséquent le déplacement des germes des parties septiques sur les zones à opérer.

Bien entendu, la description précédente n'est qu'illustrative par ses exemples et ne peut être entendue comme limitative.

## Revendications

1. Ustensile de badigeonnage destiné à l'application d'un produit pour la désinfection, la décontamination, l'aseptisation de la peau, ou tout traitement nécessitant une étape comparable, indispensable à une thérapie ou une thérapeutique nécessitant une intervention cutanée et/ou transcutanée, du type se composant d'un rouleau d'application (1) dont le moyeu creux (10), comportant des perforations (11) permettant l'arrivée dudit produit à la surface dudit rouleau (1), est monté rotatif sur un arbre creux (2) fixe et relié à un réservoir (3) dudit produit, et comportant également des perforations (9), caractérisé en ce que ledit rouleau (1) est en forme de diabolo et les perforations respectives (11,9) dudit moyeu creux (10) et dudit arbre creux (2) sont décalées les unes par rapport aux autres.

2. Ustensile de badigeonnage selon la revendication 1, caractérisé en ce que le système d'alimentation dudit rouleau comporte deux tubulures (2,2') reliées symétriquement d'une part audit réservoir (3) et d'autre part aux deux extrémités dudit moyeu creux (10).

3. Ustensile de badigeonnage selon la revendication 2, caractérisé en ce que lesdites tubulures (2,2') sont réunies audit réservoir (3) par l'intermédiaire d'une plaque de fixation (8) assurant la rigidité de l'ensemble du système.

4. Ustensile de badigeonnage selon l'une quelconque des revendications 1 à 3, caractérisé en ce que le manche creux (3) qui sert de réservoir est amovible et constitue la recharge de liquide thérapeutique pré-opératoire et comporte une capsule d'étanchéité (5) au niveau de l'arrivée du tuyau coudé (6).

5. Ustensile de badigeonnage selon l'une quelconque des revendications 1 à 3, caractérisé en ce que le manche creux (3) comporte un logement destiné à recevoir des recharges de liquide thérapeutique comprenant une capsule d'étanchéité (5) positionnée en correspondance avec l'orifice d'arrivée du tuyau coudé (6).

6. Ustensile de badigeonnage selon la revendication 5, caractérisé en ce que l'extrémité du tuyau coudé (2) relié au manche (3) comporte un moyen de perforer la capsule de sécurité (5) de la recharge lors de la phase de fixation.

7. Ustensile de badigeonnage selon la revendication 6, caractérisé en ce que le moyen de perforation consiste en un filetage (7) en bout de tuyau coudé (2) s'adaptant dans un filetage correspondant de l'orifice (6) perforant la capsule d'étanchéité au vissage après destruction de la bague circulaire (8) indiquant le contact entre la capsule et le tuyau coudé.

8. Ustensile de badigeonnage selon l'une quelconque des revendications précédentes, caractérisé en ce que le manche creux (3) comporte un capuchon clipsé ou vissé (14) dans sa partie opposée à la zone de fixation du tuyau coudé (2).

9. Ustensile de badigeonnage selon l'une quelconque des revendications précédentes, caractérisé en ce que le rouleau (1) s'adapte sur l'arbre terminal du tuyau (2) de façon à pouvoir être enlevé, au moyen de bagues de maintien amovibles (12) et (13), et des formes respectives des arbres du rouleau (1) et du tuyau coudé (2).

10. Ustensile de badigeonnage selon l'une quelconque des revendications précédentes, caractérisé en ce que l'axe du rouleau (1) est situé dans un plan différent de celui des tubulures réunissant le moyeu creux au manche-réservoir.

## Patentansprüche

1. Einreibegerät zur Auftragung eines Produkts für die Desinfektion, die Dekontamination oder die Herstellung der Asepsis der Haut oder für jede andere eine vergleichbare Stufe erfordernde Behandlung, die für eine Therapie oder für eine Therapeutik unerläßlich ist, welche einen kutanen und/oder transkutanen Eingriff erfordert, wobei das Gerät von einem Typ ist, der eine Auftragungswalze (1) umfaßt, deren Hohlnabe (10) Lochungen (11) aufweist, die ermöglichen, daß das Produkt zur Oberfläche der Walze (1) gelangt, wobei die Auftragungswalze (1) an einer festen Hohlwelle (2) drehbar angebracht ist, welche mit einem Behälter (3) für das Produkt verbunden ist und ebenfalls Lochungen (9) aufweist, dadurch gekennzeichnet, daß die Walze (1) die Form einer doppelkegeligen Rolle besitzt und die jeweiligen Lochungen (11, 9) der Hohlnabe (10) bzw. der Hohlwelle (2) zueinander versetzt sind.

2. Einreibegerät gemäß Anspruch 1, dadurch gekennzeichnet, daß das Versorgungssystem der Walze zwei Rohrstutzen (2, 2') aufweist, die symmetrisch einerseits mit dem Behälter (3) und andererseits mit den beiden Enden der Hohlnabe (10) verbunden sind.

3. Einreibegerät gemaß Anspruch 2, dadurch gekennzeichnet, daß die Rohrstutzen (2, 2') am Behälter (3) mittels eine Befestigungsplatte (8) vereinigt sind, welche die Starrheit des gesamten Systems gewährleistet.

4. Einreibegerät gemäß einem der Ansprüche 1 bis 3, dadurch gekennzeichnet, daß der als Behälter dienende hohle Handgriff (3) abnehmbar ist und die Ersatzfüllung der präoperativen therapeutischen Flüssigkeit bildet und auf Höhe der Eintritts des gekrümmten Rohrs (6) eine Dichtungskapsel (5) enthält.

5. Einreibegerät gemäß einem der Ansprüche 1 bis 3, dadurch gekennzeichnet, daß der hohle Handgriff (3) einen Aufnahmeraum aufweist, der dazu bestimmt ist, Ersatzfüllungen der Therapeutischen Flüssigkeit aufzunehmen, und eine Dichtungskapsel (5) aufweist, die in Koinzidenz mit der Eintrittsmündung der gekrümmten Rohrs (6) positioniert ist.

6. Einreibegerät gemäß Anspruch 5, dadurch gekennzeichnet, daß das mit dem Handgriff (3) verbundene Ende des gekrümmten Rohrs (2) ein Mittel aufweist, um während der Befestigungsphase die Sicherheitskapsel (5) der Ersatzfüllung zu durchstechen.

7. Einreibegerät gemäß Anspruch 6, dadurch gekennzeichnet, daß das Mittel zum Durchstechen aus einem am Ende des gekrümmten Rohrs (2) befindlichen Gewinde (7) besteht, welche an ein entsprechendes Gewinde der Mündung (6) angepaßt ist und nach der Zerstörung des kreisförmigen Rings (8), welche den Kontakt zwischen der Kapsel und dem gekrümmten Rohr anzeigt, beim Einschrauben die Dichtungskapsel durchticht.

8. Einreibegerät gemäß einem der vorangehenden Ansprüche, dadurch gekennzeichnet, daß der hohle Handgriff (3) in seinem der Zone für die Befestigung des gekrümmten Rohrs (2) gegenüberliegenden Teil eine eingerastete oder eingeschraubte Verschlußkappe (14) aufweist.

9. Einreibegerät gemäß einem der vorangehenden Ansprüche, dadurch gekennzeichnet, daß die Walze (1) an eine Endwelle des Rohrs (2) angepaßt ist, derart, daß sie mittels abnehmbarer Halteringe (12) und (13) abnehmbar ist und jeweils wie die Wellen der Walze (1) und des gekrümmten Rohrs (2) geformt ist.

10. Einreibegerät gemäß der vorangehenden Ansprüche, dadurch gekennzeichnet, daß die Mittelachse der Walze (1) in einer Ebene angeordnet ist, die von derjenigen der Rohrstutzen verschieden ist, welche die Hohlnabe am Handgriff-Behälter vereinigen.

## Claims

1. Painting implement intended for applying a product for disinfecting, decontaminating or asepticising the skin, or any treatment requiring a comparable stage, essential in any treatment or therapeutics requiring a cutaneous and/or transcutaneous intervention, of the type consisting of an application roller (1), the hollow core (10) of which, having perforations (11) enabling the said product to reach the surface of the said roller (1), is mounted so as to rotate on a fixed hollow shaft (2) connected to a reservoir (3) for the said product and also having perforations (9), characterised in that the said roller (1) is in the form of a twin roller assembly and the respective perforations (11, 9) in the said hollow core (10) and the said hollow shaft (2) are offset with respect to each other.

2. Painting implement according to Claim 1, characterised in that the system for feeding the said roller includes two tubes (2, 2') connected symmetrically on the one hand to the said reservoir (3) and on the other hand to the two ends of the said hollow core (10).

3. Painting implement according to Claim 2, characterised in that the said tubes (2, 2') are connected to the said reservoir (3) by means of a fixing plate (8) affording rigidity to the entire system.

4. Painting implement according to any one of Claims 1 to 3, characterised in that the hollow handle (3) which serves as a reservoir is removable and constitutes the preoperative therapeutic liquid refill and has a sealing cap (5) at the inlet from the angled pipe (6).

5. Painting implement according to any one of Claims 1 to 3, characterised in that the hollow handle (3) has a housing intended to receive therapeutic liquid refills comprising a sealing cap (5) positioned so as to connect with the inlet orifice of the angled pipe (6).

6. Painting implement according to Claim 5, characterised in that the end of the angled pipe (2) connected to the handle (3) has a means of perforating the safety cap (5) of the refill during the fixing stage.

7. Painting implement according to Claim 6, characterised in that the perforation means consists of a thread (7) at the end of the angled pipe (2) fitting into a corresponding tapping on the orifice (6) perforating the sealing cap during screwing after destruction of the circular ring (8) indicating contact between the cap and the angled pipe.

8. Painting implement according to any one of the preceding claims, characterised in that the hollow handle (3) has a cap (14) snapped on or screwed at its part opposite to the zone where the angled pipe (2) is fixed.

9. Painting implement according to any one of the preceding claims, characterised in that the roller (1) fits onto the end shaft of the pipe (2) in a manner which enables it to be removed, by means of removable holding rings (12) and (13), and the respective shapes of the shafts of the roller (1) and angled pipe (2).

10. Painting implement according to any one of the preceding claims, characterised in that the axis of the roller (1) is situated in a different plane from the tubes connecting the hollow core to the handle reservoir.
